# EUROPEAN PATENT APPLICATION

(11) **EP 2 554 122 A1**
(43) Date of publication of application: **06.02.2013**
(21) Application number: 11762340.5
(22) Date of filing: 02.02.2011
(51) Int. Cl.: A61B 8/00

(54) **PORTABLE ULTRASONIC DIAGNOSTIC DEVICE**

(30) Priority: 31.03.2010 JP 2010080130
(71) Applicant: Hitachi Medical Corporation, Chiyoda-ku Tokyo 101-0021 (JP)
(72) Inventor: NINOMIYA, Atsushi, Tokyo 100-8220 (JP); YANASE, Kazuyuki, Tokyo 100-8220 (JP); YOKOYAMA, Masaru, Tokyo 100-8220 (JP); KASHIWAGI, Takashi, Tokyo 101-0021 (JP); MATSUSHITA, Taisuke, Tokyo 101-0021 (JP)
(74) Representative: MERH-IP Matias Erny Reichl Hoffmann
(86) International application number: PCT/JP2011/052155
(87) International publication number: WO 2011/122099

(57) **Abstract**

A small-sized ultrasonic diagnostic device is provided which is capable of taking various storage positions, and good in usability. The portable ultrasonic diagnostic device includes a main body 30, a keyboard body 50, and a display body 80, wherein the keyboard body 50 is provided with an input operation key layout surface 51 on the top face thereof and coupled to the main body 30 in a rotatable manner, via a first pivot part 100 provided on a rear end of the keyboard body, and the display body 80 is provided with a display screen 81 on a front face thereof and coupled to the main body 30 in a rotatable manner via a second pivot part 200 provided on a lower end of the display body.

## Description

### Technical Field

The present invention relates to a portable ultrasonic diagnostic device with superior installability.

### Background Art

A conventional ultrasonic diagnostic device is a wagon type, in most cases, being a movable carriage on which various devices are mounted, but a portable ultrasonic diagnostic device with a superior portability is now made in public. By way of example, a portable ultrasonic diagnostic device referred to as a notebook type, has a structure that a lid type body equipped with a display unit is foldable into a thin-type main body. Another portable ultrasonic diagnostic device referred to as a vertical type, is provided with a display unit on the front face of the main body being narrow in depth, and a foldable keyboard is provided on the body surface in the lower position of the display unit. Accordingly, in the usage state, the keyboard is open toward the front of the display unit for allowing input operation, and in the non-usage state, the keyboard is foldable in such a manner as covering over the display unit. In order to enhance visibility of the display unit, there is suggested one of such vertical type devices, provided with a tilt mechanism for the display unit, enabling vertical angle variation.

### Prior Art Document

### Patent Document

[Patent Document 1]
   Japanese Unexamined Patent Application Publication No. 8-252250
[Patent Document 1]
   Japanese Unexamined Patent Application Publication No. 5-19895
[Patent Document 1]
   Japanese Unexamined Patent Application Publication No. 5-53685
[Patent Document 1]
   Japanese Unexamined Patent Application Publication No. 9-244763

### Disclosure of the Invention

### Problem to be solved by the Invention

As for the conventional notebook type example, it is possible to take on a briefcase-like shape when it is transported (carried), but in the usage state, the largest plane of the main body is supposed to be placed on a table surface for the usage. Therefore, there is a problem that a large installation area required for any of the states; working thereon and leaving it placed (stored). Furthermore, in some cases, the ultrasonic diagnostic device is required to show a diagnostic image to a patient, but the conventional notebook type device is not provided with a function for allowing the display unit to swivel.

As for the conventional vertical type example, the main body takes on an upright shape, and thus its installation area is small. Therefore, it is advantageous that in the stored state under the non-usage condition, the keyboard is folded and the surface of a table, or the like, is widely available, but on the other hand, it is poor in portability. In addition, there is a problem that in the vertical type, the main body is equipped with the display unit, and thus this makes it difficult to provide the function for swiveling.

In other words, any of the conventional portable ultrasonic diagnostic devices is usable with the keyboard being placed at the front of the display unit in the usage state, but they do not fulfill requirements as the ultrasonic diagnostic device, i.e., the function for allowing the display unit to swivel and the portability.

In view of those described above, an object of the present invention is to provide a portable ultrasonic diagnostic device which is able to take various storage positions and has excellent usability.

### Means to solve the Problem

In order to achieve the object described above, the ultrasonic diagnostic device relating to the present invention is provided with a main body, a keyboard body, and a display body, wherein the keyboard body is provided with an input operation key layout on a top face thereof and coupled to the main body in a rotatable manner via a first pivot part provided on a rear end of the keyboard body, and the display body is provided with a display screen on a front face thereof and coupled to the main body in a rotatable manner via a second pivot part provided on a lower end of the display body.

### Effect of the Invention

According to the portable ultrasonic diagnostic device of the present invention, it is possible to take various storage positions, and enhance the usability.

### Brief Description of Drawings

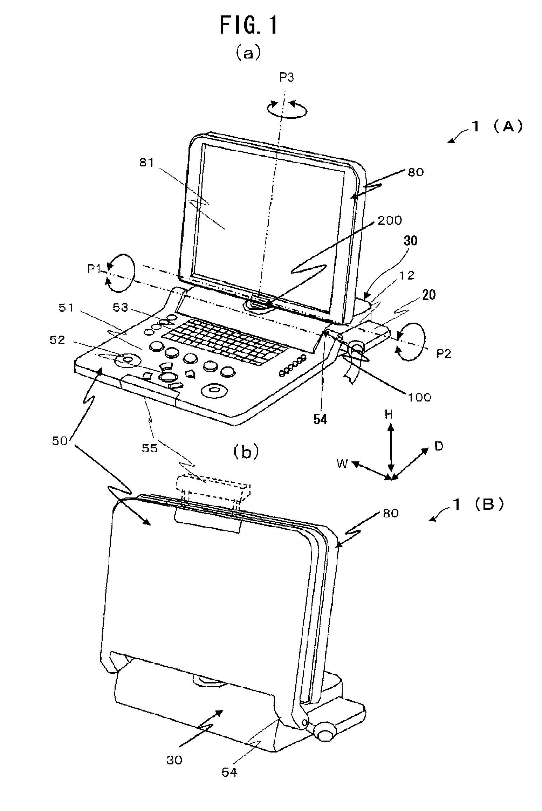

FIG. 1 is a schematic view of the portable ultrasonic diagnostic device relating to a first embodiment of the present invention; FIG. 1 (a) is a perspective view of the portable ultrasonic diagnostic device 1, taking an operating position A where the keyboard body 50 maintains nearly horizontal position and the display body 80 is made to stand, and FIG. 1(b) is a perspective view of the portable ultrasonic diagnostic device 1, taking a first storage position B where the keyboard body 50 is made to stand up from the operating position A;

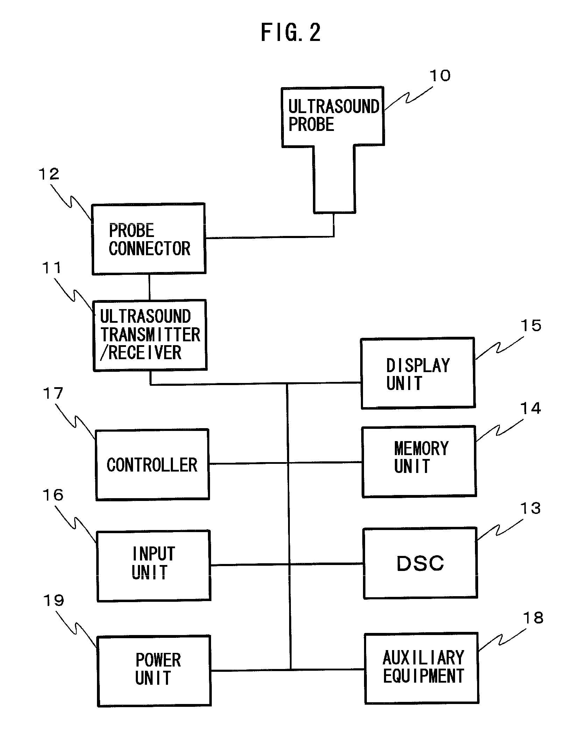

FIG. 2 is a device block diagram showing the portable ultrasonic diagnostic device relating to the first embodiment of the present invention;

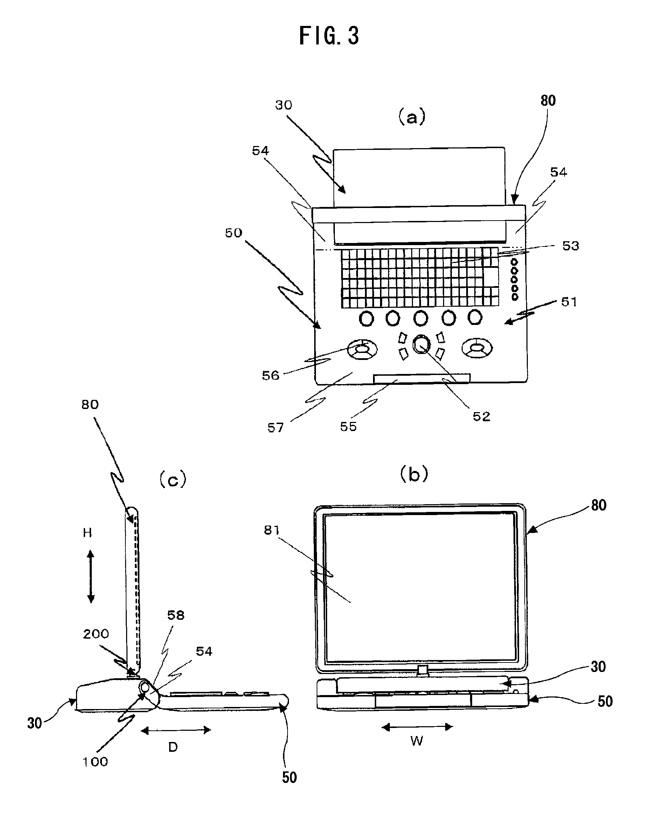

FIG. 3 is an external view of the portable ultrasonic diagnostic device relating to the first embodiment of the present invention; FIG. 3 (a) is a top view of the portable ultrasonic diagnostic device taking the operating position A; FIG. 3(b) is a front view thereof, and FIG. 3(c) is a side view thereof;

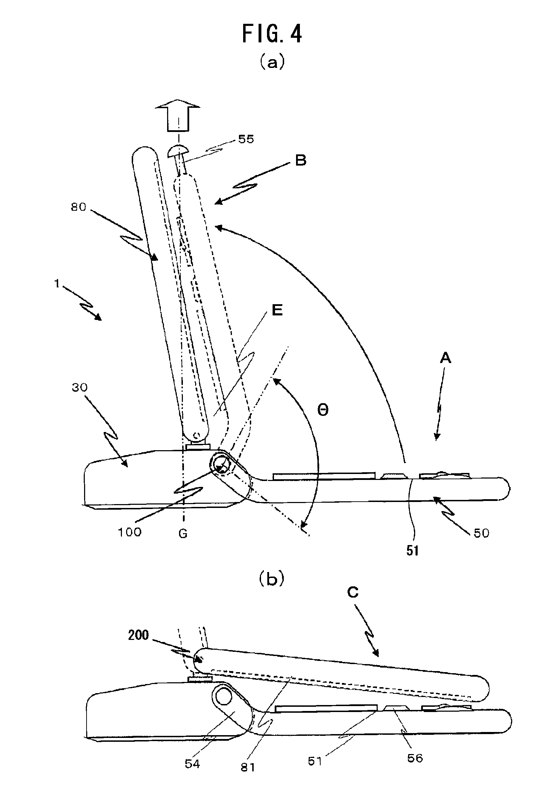

FIG. 4 illustrates a moving state of the display body and the keyboard body of the portable ultrasonic diagnostic device relating to the first embodiment of the present invention; FIG. 4 (a) is a side view showing variation from the operating position A to the first storage position B, and FIG. 4(b) is a side view of the portable ultrasonic diagnostic device taking the second storage position C in which the display body 80 is folded down to the front side from the operating position A;

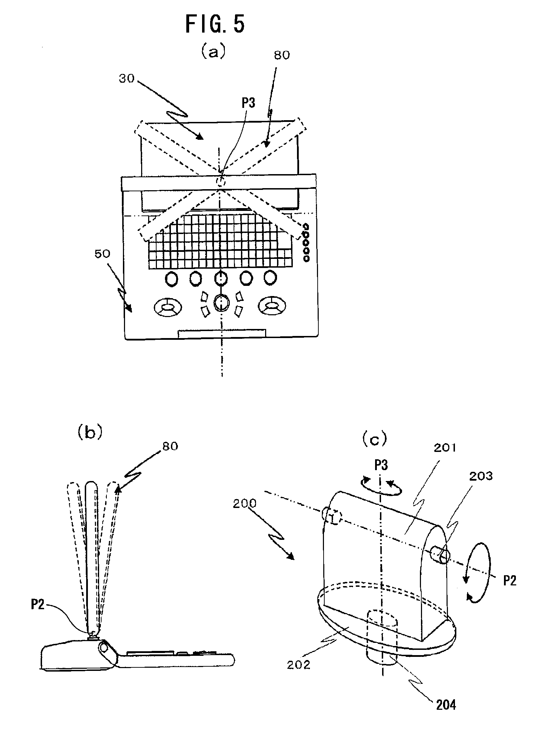

FIG. 5 illustrates the moving state of the display body of the portable ultrasonic diagnostic device relating to the first embodiment of the present invention; FIG. 5(a) is a top view showing the state of the display body 80 swiveling in the operating position A, FIG. 5 (b) is a side view showing the state that the display body 80 tilts in the operating position A, and FIG. 5 (c) is a schematic diagram of a second pivot part 200;

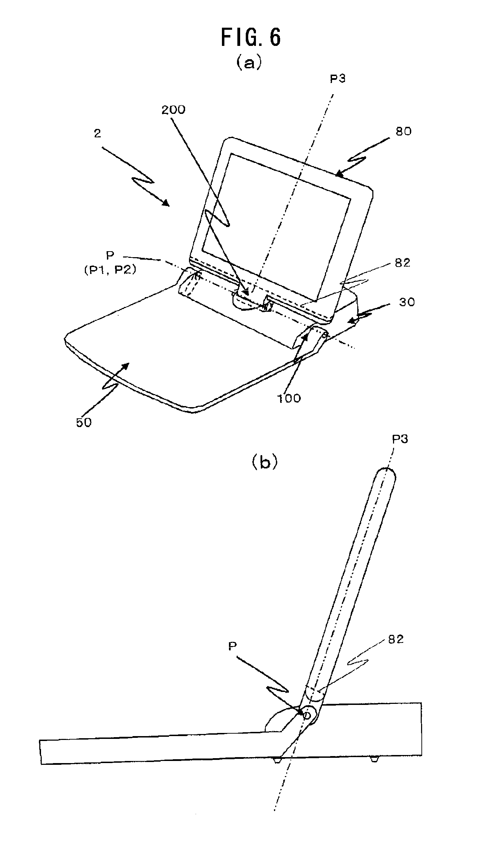

FIG. 6 is an external view of the portable ultrasonic diagnostic device relating to a second embodiment of the present invention; FIG. 6(a) is an external perspective view in the usage state, and FIG. 6(b) is a side view thereof;

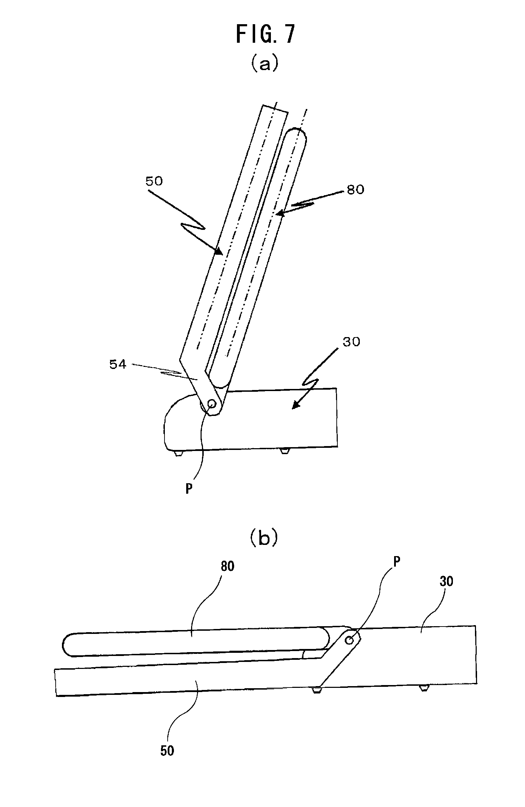

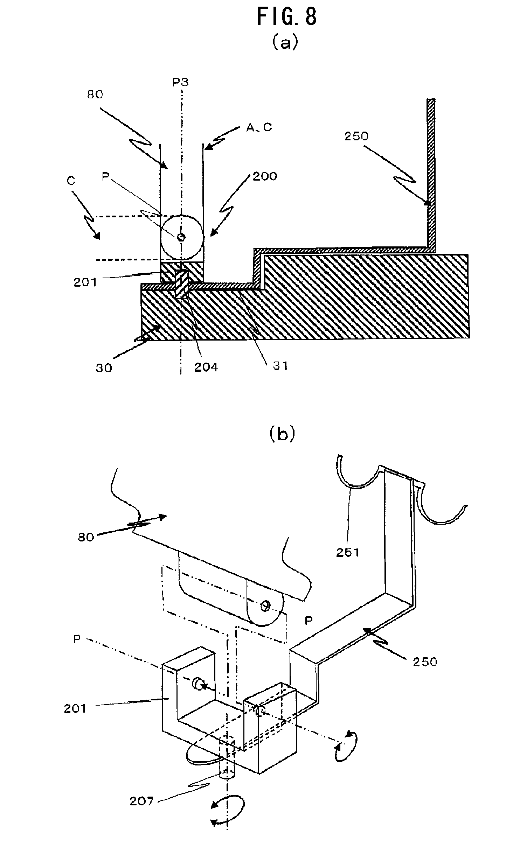

FIG. 7 illustrates the moving state of the portable ultrasonic diagnostic device relating to the second embodiment of the present invention, FIG. 7(a) is a side view of the first storage position B, and FIG. 7 (b) is a side view of the second storage position C; FIG. 8 is a detail view of an alternative application example of the portable ultrasonic diagnostic device relating to the second embodiment of the present invention; FIG. 8 (a) is a cross-sectional view of the second pivot part, and FIG. 8 (b) is a development view of the second pivot part;

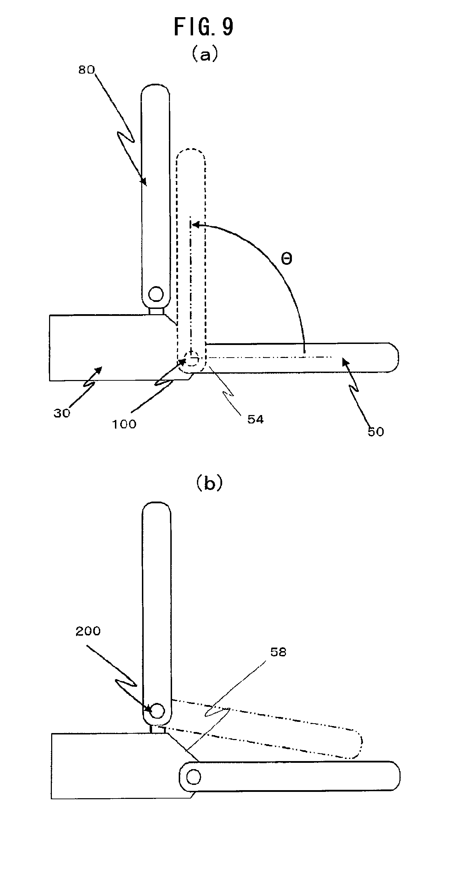

FIG. 9 illustrates the moving state of the display body and the keyboard body of the portable ultrasonic diagnostic device relating to the alternative application example of the present invention; FIG. 9(a) is a side view showing the state changing from the operating position A to the first storage position B, and FIG. 9(b) is a side view showing the state changing from the operating position A to the second storage position C; and

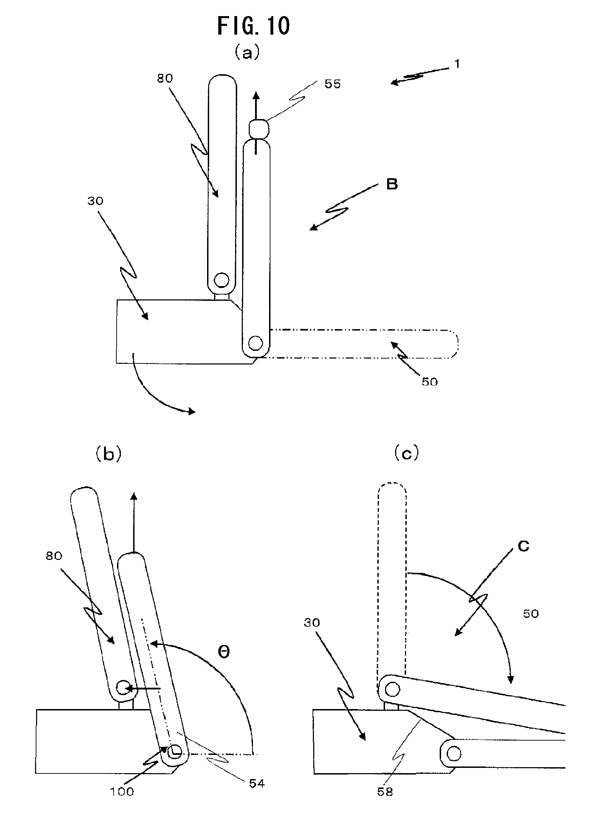

FIG. 10 illustrates the moving state of the display body and the keyboard body of the portable ultrasonic diagnostic device relating to the alternative application example of the present invention; FIG. 10 (a) and FIG. 10 (b) are side views showing the state changing from the operating position A to the first storage position B, and FIG. 10(c) is a side view showing the state changing from the operating position A to the second storage position C.

### Modes for Carrying Out the Invention

Hereinafter, with reference to FIG. 1 to FIG. 10, the portable ultrasonic diagnostic device relating to the present invention will be explained concretely. Here, the drawings from FIG. 1 to FIG. 5 illustrate the portable ultrasonic diagnostic device relating to the first embodiment, the drawings from FIG. 6 to FIG. 8 illustrate the portable ultrasonic diagnostic device relating to the second embodiment, and the drawings of FIG. 9 and FIG. 10 illustrate the portable ultrasonic diagnostic device relating to the alternative application example. It is to be noted that similar portions, arrows, and the like, are labeled the same, and tedious explanations will not be made.

### First Embodiment

Firstly, with reference to FIG. 1, a schematic structure of the portable ultrasonic diagnostic device 1 relating to the first embodiment will be explained. FIG. 1(a) is a perspective view of the portable ultrasonic diagnostic device 1, taking the operating position A in which the keyboard body 50 takes nearly horizontal position and the display body 80 is made to stand, and FIG. 1(b) is a perspective view of the portable ultrasonic diagnostic device 1, taking the first storage position B in which the keyboard body 50 is raised to stand up, from the operating position A.

In FIG. 1, the portable ultrasonic diagnostic device collectively represented by the reference numeral 1, is made up bodies including a main body 30 provided with principal functions of the ultrasonic diagnostic device, a keyboard body 50 mounted on the main body 30 in a rotatable manner via a first pivot part 100, and a display body 80 mounted on the main body 30 in a rotatable manner via a second pivot part 200.

The main body 30 has a flat appearance configuration that the depth dimension D is larger than the height dimension H, and the width dimension W is larger than the depth dimension D. The keyboard body 50 has a flat appearance configuration that the depth dimension D is larger than the height dimension H, and the width dimension W is larger than the depth dimension D. The display body 80 has an appearance configuration that the height dimension H is larger than the depth dimension D, and the width dimension W is larger than the height dimension H.

In addition, on the keyboard body 50, there is placed an input operation key layout surface 51 provided with a trackball 52 and a keyboard part 53 enabling a character input, on the top face which forms the widest surface of the keyboard body. On the rear end of the keyboard 50, there are formed arm parts 54, both sides extending backward, and the arm parts 54 are coupled to the front section of the main body 30 in a rotatable manner, via the first pivot part 100 being provided on the pair of the arm parts 54.

On the other hand, the display body 80 is provided with a display screen 81 on the front face which forms the widest surface of the display body. The second pivot part 200 is provided on the lower end of the display body 80, and it is coupled to the upper front section of the main body 30 in rotatable manner, via the second pivot part 200.

One of quite characteristic points of the small-sized ultrasonic diagnostic device 1 relating to the present embodiment is that this ultrasonic diagnostic device employs the following structure; it is able to take the operating position A (FIG. 1(a)) in which the keyboard body 50 takes a nearly horizontal position and the display body 80 is made to stand, the first storage position B (FIG. 1(b)) with a small installation area where the keyboard body 50 is raised to stand up from the operating position A, and the second storage position C (FIG. 4(b)) in which the display body 80 is folded forward to be low in height, from the operating position A. It is to be noted that the nearly horizontal position indicates a posture being almost horizontal.

In other words, it is a matter of course that the conventional portable ultrasonic diagnostic device takes an operating position, where the keyboard takes the nearly horizontal position and the display body takes the standing position. However, in the storage position changed from this operating position, the conventional portable ultrasonic diagnostic device has a structure which allows only either one of the following positions; the storage position with a small installation area, and the storage position sacrificing the height. By way of example, as for the notebook type, it has a structure that only the display body is movable. Therefore, it is possible to perform storage in a position being flat with respect to the installation area, whereas it requires a large installation area. On the other hand, as for the vertical type, it has a structure to fold the keyboard body up toward the vertical type main body. Therefore, though it is possible to take a storage position which requires just a small installation area, this storage position is voluminous, caused by standing up on a large scale from the installation surface, and thus it is not good in portability.

In view of those described above, the portable ultrasonic diagnostic device 1 relating to the present embodiment employs a structure which is able to take any of the storage positions; the notebook type and the vertical type, thereby achieving various storage positions.

In other words, the first pivot part 100 of the present embodiment has a structure provided with a pivot P1 for holding the keyboard body 50 in such a manner that it is allowed to take a standing position on the top of the main body 30, from the nearly horizontal position at the front of the main body 30, and the second pivot part 200 has a structure provided with a pivot P2 for holding the display body 80 in such a manner as taking a nearly horizontal position covering the top of the keyboard body 50 which takes the nearly horizontal position, from the standing position on the top of the main body 30.

With this structure, the keyboard body 50 is raised and takes a standing position nearly parallel with the display body 80, as shown in FIG. 1(b), from the operating position A as shown in FIG. 1(a), thereby achieving the first storage position B which requires only a small installation area, similar to the conventional vertical type. On the other hand, in order to take the second storage position C which is low in height, the position where the display body 80 is folded forward, as shown in FIG. 4(b), from the operating position A as shown in FIG. 1(a), thereby achieving the second storage position C being low in height, similar to the conventional notebook type.

In addition, according to the present embodiment, the first storage position B as shown in FIG. 1(b) allows for a compact storage of the display body 80 and the keyboard body 50 on the top of the main body 30. Therefore, it is possible to transport (carry) the portable ultrasonic diagnostic device 1, in the similar manner as the conventional vertical type. Further in the present embodiment, there is provided a lock mechanism, not illustrated, for fixing the rotation angle at the first pivot part 100 and the second pivot part 200. Accordingly, the first storage position B is secured, thereby achieving a good carrying property in this first storage position B.

One of the other quite characteristic points of the small-sized ultrasonic diagnostic device 1 relating to the present embodiment is that a handle part 55 is provided in the front section of the keyboard body 50 (operating position A). With this handle part 55 being provided, when the first storage position B is taken as shown in FIG. 1(b), this handle part 55 is located in the upper section and a user is allowed to hold the handle part 55 and carry the portable ultrasonic diagnostic device 1 in the similar manner as the conventional vertical type. Further in the second storage position C as shown in FIG. 4(b), the user is allowed to hold the handle part 55 and carry the portable ultrasonic diagnostic device 1 in the similar manner as the conventional notebook type.

One of the other quite characteristic points of the small-sized ultrasonic diagnostic device 1 relating to the present embodiment is that a pivot P3 for allowing the display body 80 to swivel is provided on the second pivot part 200. In other words, the present embodiment has a structure that the display body 80 and the main body 30 are coupled via the second pivot part 200 being mounted at the center of the lower end of the display body 80, the second pivot part 200 being provided with the pivot P1 allowing the display body 80 to be folded forward, and the pivot P3 allowing the display body 80 to swivel. With this configuration, in the operating position A, the display body 80 can swivel on the pivot P3 in the operating position A, thereby enhancing the usability as the ultrasonic diagnostic device.

One of the other quite characteristic points of the small-sized ultrasonic diagnostic device 1 relating to the present embodiment is a structure that a pair of the arm parts 54 are curved upwardly with respect to the depth direction of the keyboard body 50.

In other words, in the present embodiment, as shown in FIG. 1(b), when the first storage position B is taken, the keyboard body 50 and the display body 80 in the standing position are stored, in such a manner as biased to the front side above the main body 30. Therefore, there are problems that in the first storage position B, the standing keyboard body 50 may build a feeling of pressure for the user, while in the carrying position via the handle part 55, the center of gravity is biased, making the carriage difficult. Those problems may be mitigated by enlarging the turning range of the keyboard body 50, and bringing the keyboard body down to the main body side. However, if the turning range of the keyboard body 50 is enlarged, it is necessary to elongate the length of the arm parts 54, causing another problem that the structure in the vicinity of the second pivot part 200 may grow in size.

Considering the situation above, in the present embodiment, the pivot P1 of the first pivot part 100 is provided at a higher position in the front section of the main body 30, the pivot P1 at this higher position and the keyboard body 50 in the nearly horizontal position are coupled via the arm part 54 being curved, thereby mitigating the problem above. Further with this structure, in the first storage position B as shown in FIG. 4(a), it is possible to form a gap E between the keyboard body 50 and the display body 80. By the use of this gap, it is possible to prevent the input operation keys having a certain height placed in the input operation key layout surface 51, from coming into contact with the display body 80, and this contributes to downsizing. In particular, this is advantageous in the portable ultrasonic diagnostic device 1, since it requires to have the input operation keys with a certain height, which is not employed in a general personal computer.

Hereinafter, a further detailed explanation will be made as to a specific structure of the portable ultrasonic diagnostic device 1 relating to the first embodiment, provided with the aforementioned characteristics.

Firstly, with reference to FIG. 2, a device configuration of the portable ultrasonic diagnostic device 1 will be explained. FIG. 2 is a device block diagram showing the portable ultrasonic diagnostic device 1. In FIG. 2, the portable ultrasonic diagnostic device 1 incorporates an ultrasound probe 10 provided with a group of ultrasound transducers, an ultrasound transmitter/receiver 11 for supplying a high-voltage pulse to the ultrasound probe 10, a probe connector 12 for establishing connection between the ultrasound transmitter/receiver 11 and the ultrasound probe 10, a digital scan converter 13 (hereinafter, referred to as DSC) for converting an echo into a digital signal, a memory unit 14 made up of an image memory, a shared graphics memory, an operator-use graphics memory, and the like, a display unit 15 provided with the display screen 81, an input unit 16 made up of the trackball 52 and the keyboard part 53, a controller 17 for performing overall control over the portable ultrasonic diagnostic device 1, and auxiliary equipment 18 such as an electrocardiogram (ECG) measuring unit being connected as required, and a power unit 19 for supplying power to each of the units above.

The ultrasound transmitter/receiver 11 supplies a high-voltage pulse for wave transmission to the group of ultrasound transducers within the ultrasound probe 10. In addition, the ultrasound transmitter/receiver 11 amplifies, performs phasing of, and detects the echoes being reflected from inside of a subject body and received by the group of transducers, and inputs the outputted signal into the DSC 13. The ultrasound probe 10 has a cord of a predetermined length, and it is connected to the probe connector 12 which is mounted in association with the ultrasound probe 10, via a cord connection part 20 (see FIG. 1) provided on the end of the cord.

The DSC 13 converts the signal received from the ultrasound probe 10 into a digital signal, every time the transmission and receipt of an ultrasound beam one by one are repeated and writes the digital signal into the memory. In addition, the DSC 13 reads out memory contents in sync with scanning on the display unit 15. The shared graphics memory constituting the memory unit 14, is a graphics memory for displaying additional information (information not bringing a sense of anxiety to the subject, e.g., a scale of an ultrasound image, a body mark indicating a position where the ultrasound probe 10 abuts against the subject, and distance estimation information), the additional information being displayed in such a manner as superimposing on an ultrasound image that is displayed on the display unit 15 via the DSC 13. The operation-use graphics memory of the memory unit 14 is a graphics memory for displaying in such a manner as superimposing on the ultrasound image on the display unit 15, only the additional information (e.g., character information such as a disease name, ID information of the subject, and symbol information such as an arrow indicating a site of lesion) which is required for an operator to conduct diagnosis.

According to this ultrasonic diagnostic device 1, the input unit 16 is manipulated to configure settings of an ultrasound scanning mode and a display of additional measurement information. The ultrasound probe 10 is made to abut against a site to be inspected on the subject, and then the inspection is started. The ultrasound wave transmitted into the body of the subject from the ultrasound probe 10 is reflected by a boundary having a different acoustic impedance in the internal organ, and received by the probe. The ultrasound transmitter/receiver 11 amplifies, performs phasing of, and detects the echoes being received. The detected signals are inputted into the DSC 13 as signals corresponding to one ultrasound scanning line, subjected to D/A conversion and written into the memory. Writing into the memory is carried out upon every repetition of transmitting and receiving, along with the change of the direction for ultrasound transmission and receipt.

The image data written into the memory is read out in sync with display scanning on the display unit 15, subjected to D/A conversion, supplied to the display unit 15 as brightness signals. As a result, an ultrasound image is displayed on the screen. In sync with reading of the image data from the DSC 13, the controller 17 reads data both from the shared graphics memory and the operator-use graphics memory. In the present embodiment, upon showing the display screen of the display unit 15 to the subject, it is possible to configure such that the contents in the operator-use graphics memory are made invisible. Switching of the display screens may be manipulated via the input unit 16.

Next, with reference to FIG. 1, and FIG. 3 from FIG. 5, an explanation will be made as to an external structure of the portable ultrasonic diagnostic device 1 relating to the present embodiment. Here, FIG. 3 (a) is a top view of the portable ultrasonic diagnostic device in the operating position A, FIG. 3 (b) is a front view thereof, and FIG. 3(c) is a side view thereof. FIG. 4 illustrates a moving state of the display body 80 and the keyboard body 50 of the portable ultrasonic diagnostic device 1; FIG. 4 (a) is a side view showing variation from the operating position A to the first storage position B, and FIG. 4(b) is a side view of the portable ultrasonic diagnostic device 1, where the display body 80 is folded toward the front side, from the operating position A, so as to take the second storage position C. FIG. 5 illustrates the moving state of the display body of the portable ultrasonic diagnostic device; FIG. 5 (a) is a top view showing the state of the display body 80 swiveling in the operating position A, FIG. 5 (b) is a side view showing the state that the display body 80 tilts in the operating position A, and FIG. 5(c) is a schematic diagram of the second pivot part 200.

Firstly, as obvious from FIG. 1 and FIG. 3, in the present embodiment, the width dimension W of the main body 30 is made smaller than the width dimension W of the keyboard body 50, and a surplus portion of the width dimension W of the keyboard 50 relative to the width of the main body 30 is equally allocated on both sides of the main body 30, thereby forming the arm parts 54. Accordingly, it is structured such that the main body 30 is coupled to the arm parts 54, in such a manner as placing the front section of the main body 30 between one pair of the arm parts 54. It is a matter of course the arm parts 54 may be concentrated to one side, allowing one arm part to couple the main body 30 to the keyboard body 50.

The pair of the arm parts 54 extending toward the rear side are formed in such a manner as being curved upwardly with respect to the keyboard body 50 in the nearly horizontal position. With this configuration, it is possible to set the first pivot part 100 at a higher position. In other words, the first pivot part 100 is placed at a higher position in the front section of the main body 30 on both side-surfaces thereof.

The input operation key layout surface 51 of the keyboard body 50 incorporates the keyboard part 53 enabling the character input in the rear section thereof, an operation key group 56 in the front section thereof, placing the trackball 52 at the center, and further preserves a flat surface at the front of the operation key group, thereby forming a palm rest part 57. The palm rest part 57 supports wrists of both hands or a wrist of one hand, thereby enabling input operation on the operation key group 56 and the keyboard part 53.

The front edge of the palm rest part 57 is provided with the handle part 55 which is able to be pulled out forwardly. This handle part 55 is retractable into the keyboard body 50 at the time of inputting operation or at the time of storage, whereas the handle part 55 is able to be pulled out for use, when the ultrasonic diagnostic device 1 is carried.

Though not illustrated, it is further possible to provide another palm rest part which is pulled out from the keyboard body 50. The palm rest part which is able to be pulled out is configured in such a manner that it is stored inside the keyboard body 50 and pulled out therefrom. The operator draws this palm rest part which is able to be pulled out, thereby supporting wrists of both hands or a wrist of one hand, and enabling inputting operation on the operation key group 56 or on the keyboard part 53.

On the other hand, the display body 80 is coupled to the main body 30, via the second pivot part 200 that is arranged on the top of the main body 30 at the center in the front section thereof. The width dimension W of this display body 80 is a size corresponding to the width dimension W of the keyboard body 50, as shown in FIG. 3(a) and (b). The second pivot part 200 for supporting the display body 80 is provided, being displaced toward the rear side relative to the position for mounting the first pivot part 100, in order to reserve the movable range of the keyboard body 50. The upper edge part 58 in the front section of the main body 30 is formed as an inclined surface with the edge being cut off, or a large arc-like surface, in order to reserve the movable range of the keyboard body 50 and the display body 80. With this configuration, it is possible to reserve the aforementioned movable range and keep the length of the arm parts 54 to the minimum. In addition, since the portion around the user's line of sight has a shape being rounded off, this gives the impression that the apparatus is downsized and enhances designing property.

Though not illustrated in FIG. 3(a) to (c), it is to be noted that the probe connector 12 is provided on the right side surface of the main body 30, for mounting the cord connection part 20 of the ultrasound probe 10, and further on the back surface of the main body 30, there are provided a terminal connection, a part for mounting a power supply cord, and the like, though not illustrated, for establishing connection with various equipment.

According to the portable ultrasonic diagnostic device 1 relating to the present embodiment, it is possible to take various positions as the operating position and the storage position. With reference to FIG. 4 and FIG. 5, further explanations will be made regarding this point.

As shown in FIG. 4(a), according to the present embodiment, it is possible to take a very general operating position A in which the keyboard body 50 is located in the nearly horizontal position at the front of the main body 30, and the display body 80 is located at the rear end of the keyboard body 50 in the nearly vertical position rather inclined backward. This operating position A enables a position which is good for operation.

Then, from this operating position A, the keyboard body 50 is raised, pivoting on the first pivot part 100, thereby achieving the first storage position B as indicated by the broken line. Here, in the present embodiment, the first pivot part 100 is provided with a well-known lock mechanism not illustrated, therefore, this allows the keyboard body 50 to be fixed in the horizontal state as indicated by the solid line or to stop pivoting in the standing state as indicated by the broken line, as shown in FIG. 4(a). The first pivot part 100 is also provided with a lock release mechanism against the lock mechanism. It is to be noted that the nearly vertical position indicates a posture being almost vertical.

Here, for the keyboard body 50 of the present embodiment, the arm parts 54 are curved with respect to the input operation key layout surface 51. Therefore, in the state where the keyboard body 50 stands up, it is possible to take the first storage position B where the keyboard body 50 is inclined inwardly, without enlarging the rotation angle θ of the keyboard body 50. By way of example, in the present embodiment, the rotation angle θ is set to be 90 degrees, and it is possible to achieve the position where the keyboard body 50 is inclined inwardly. In other words, this facilitates the keyboard body 50 to be placed within the projected area of the main body 30. This configuration facilitates the handle part 55 provided on the keyboard body 50 to be located in proximity to the upper side of the center of gravity G of the portable ultrasonic diagnostic device 1, and therefore, it is possible to perform preferable operation for carrying the portable ultrasonic diagnostic device 1, with holding the handle part 55.

In the first storage position B, the keyboard body 50 takes a shape being curved, thereby forming the gap E between the keyboard body 50 and the display body 80. Accordingly, the use of this gap E facilitates the operation key group 56 being of a certain height to be mounted on the input operation key layout surface 51. This effect is advantageous in the portable ultrasonic diagnostic device 1 which needs to mount the operation key group 56 being of a certain height.

On the other hand, as shown in FIG. 4(b), in the present embodiment, the display body 80 in the operating position A as indicated by the broken line is folded toward the front side, thereby allowing the display body to take the second storage position C as indicated by the solid line. The second pivot part 200 for enabling this variation is also provided with the lock mechanism and the release mechanism, in the similar manner as the first pivot part 100. In addition, fixing at an optional angle is also available. With this configuration, the display body 80 is folded from the operating position A, and accordingly the input operation key layout surface 51 and the display screen 81 can be covered. Therefore, it is possible to temporarily cover the displayed information when leaving seat, or the like, or prevent an erroneous operation. In addition, the second storage position C achieves a low-height storage position.

As shown in FIG. 5(a) and (b), in the present embodiment, the second pivot part 200 is provided with the pivot P2 enabling the display body 80 to tilt and to be folded, and a pivot P3 enabling the display body 80 to swivel. FIG. 5(c) illustrates one example of the second pivot part 200 which enables these two movements. In FIG. 5(c), the second pivot part 200 is made up of a pivot main body 201 which stands upright and the front end thereof is rounded, and a disc-like part 202 which is formed on the bottom of the pivot main body 201. On the both sides of the pivot main body 201, there are provided a pair of horizontal pivot parts 203 extending from both sides, and there is provided a vertical pivot part 204 at the center of the bottom surface of the disc-like part 202.

Then, pivot bearing parts provided on the display body 80, not illustrated, are mounted and fixed on the pair of horizontal pivot parts 203. The vertical pivot part 204 is mounted and fixed on a pivot bearing part, not illustrated, provided on the main body 203. With this configuration, it is possible that the pair of the horizontal pivot parts 203 constitute the pivot P2, and the vertical pivot part 204 constitutes the pivot P3. The display body 80 turns on the pivot P3, varying the display angle of the display body 80, and this allows a patient around the portable ultrasonic diagnostic device 1 to take a look of a diagnostic image.

By employing the second pivot part 200 having such a structure as described above, the display body 80 is allowed to turn on the pivot P3 as shown in FIG. 5(a). In addition to the folding operation of the display body 80 as shown in FIG. 4(b), a tilt operation of the display body 80 is possible forward and backward as shown in FIG. 5(b), and the display screen 81 is adjusted to be at an optional angle, thereby enhancing visibility.

### Second Embodiment

Next, with reference to FIG. 6 to FIG. 8, a specific explanation will be made as to the portable ultrasonic diagnostic device relating to the second embodiment of the present invention. FIG. 6 is an external view of the portable ultrasonic diagnostic device relating to the second embodiment of the present invention; FIG. 6(a) is an external perspective view in the usage state, and FIG. 6 (b) is a side view thereof. FIG. 7 illustrates the moving state of the portable ultrasonic diagnostic device relating to the second embodiment of the present invention, FIG. 7(a) is a side view of the first storage position B, and FIG. 7 (b) is a side view of the second storage position C. FIG. 8 is a detail view of an alternative application example of the portable ultrasonic diagnostic device relating to the second embodiment of the present invention; FIG. 8 (a) is a cross-sectional view of the second pivot part, and FIG. 8 (b) is a development view of the second pivot part.

It is to be noted that identical portions, arrows, and the like, are labeled the same, and tedious explanations will not be made. Further in the following descriptions, only the points different from the first embodiment will be explained, and the points not explained have the same structures as the first embodiment.

In FIG. 6, the portable ultrasonic diagnostic device 2 relating to the second embodiment incorporates, similar to the first embodiment, the main body 30, the keyboard body 50 rotatably mounted on the main body 30 via the first pivot part 100, and the display body 80 rotatably mounted on the main body 30 via the second pivot part 200. One of quite characteristic points of the present embodiment is that the pivot P1 of the first pivot part 100 for turning the keyboard body 50 and the pivot P2 of the second pivot part 200 for turning the display body 80 are placed on the same pivot P.

In other words, in the structure of the first embodiment where the pivot P1 and the pivot P2 do not exist on the same pivot P, there is caused a new problem that when the keyboard body 50 and the display body 80 are made to turn simultaneously, their contact point may scrape against each other, though this problem does not occur if the two bodies turn independently. This new problem may be solved by avoiding the contact between the two bodies, but increase in strength can be achieved by the contact between the two bodies. Accordingly, in the present embodiment, the pivot P1 and the pivot P2 are arranged on the same pivot P. With this configuration, it is possible to solve the aforementioned problem, in addition to the operation effect similar to that of the first embodiment.

Further as shown in FIG. 7, one of the other quite characteristic points of the second embodiment is that the longitudinal direction of the keyboard body 50 and that of the display body 80 are placed nearly in parallel with each other, in the state where the keyboard body 50 and the display body 80 are superimposed one upon another. With this configuration, even though those two bodies come into contact with each other, or one placed in the vicinity of another, all over the contact plane or the vicinal plane is subject to stress from the outside, and therefore, it is advantageous structurally. In addition, those in the state of being superimposed are able to be compactly packed together, and therefore it is good in design property.

In order to place the two bodies having the common pivot P in nearly parallel, the arm parts 54 of one of the two bodies, i.e., of the keyboard body 50 in this example here, have a shape being bent with respect to the longitudinal direction thereof. With this configuration, it is possible to obtain the aforementioned operation effect, in addition to the same operation effect as that of the first embodiment.

As shown in FIG. 6, also in the second embodiment, the main body 30 provided with the second pivot part 200 and the display body 80 provided with the display screen 81 are rotatably coupled via the pivot P3, at the position of division part 82 as indicated by the broken line, thereby achieving the same operation effect as that of the first embodiment.

As shown in FIG. 8 (a) and (b), in the alternative application example of the second embodiment, the pivot main body 201 constituting the second pivot part 200 is coupled to the main body 30 at the pivot P3, and coupled to the display body 80 at the pivot P, whereby the display body 80 is able to take the operating position A, the first storage position B, and the second storage position C, and further the display body 80 is able to swivel in the operating position A. In addition, with the coupling structure as described above, it is possible to mount on the portable ultrasonic diagnostic device 2, an accessory mount 250 for mounting accessories such as the ultrasound probe, which turns together with the swiveling operation of the display body 80.

FIG. 8 (a) and (b) illustrate one example of this detailed structure of this accessory mount 250. A concave portion 31 which permits the turning of the pivot main body 201 is formed on the main body 30, and one end of the accessory mount 250 is rotatably mounted on the vertical pivot part 204 which is provided on this concave portion 31. The accessory mount 250 is formed in a band-like shape made of a metallic material, and it is formed in such a manner as extending backwardly from the concave portion 31 along the upper surface of the main body 30. Then, the rear end of the accessory mount 250 is formed in such a manner as standing up within the projected area of the main body 30, and on the upper end thereof, there is provided a hook 251 for putting the ultrasound probe and a cord thereof.

With this configuration, in the normal state where the display body 80 is not made to swivel (FIG. 6(a) and FIG. 7(a)), the accessory mount 250 is provided in such a manner as adjacent to the rear of the display body 80, and therefore, it is possible to prepare for putting the ultrasound probe and the cord thereof on the accessory mount 250. Generally, the portable ultrasonic diagnostic device 2 sometimes uses multiple ultrasound probes, and in this embodiment, it is possible to prepare multiple ultrasound probes without posing a problem for their operation, and within an accessible operation range.

In addition, if the accessory mount 250 is provided in a secured manner, the display body 80 may come into contact with the accessories put on the accessory mount 250, when the display body 80 is made to swivel, and therefore there is a concern that those accessories may be dropped. However, in the present embodiment, since the accessory mount 250 turns on the pivot P3 of the display body 80, even though the display body 80 comes into contact with the accessory mount 250, the accessory mount 250 also turns and absorbs the shock of the contact, thereby solving the concern above.

### Alternative Application Example

Next, with reference to FIG. 9 and FIG. 10, an explanation will be made as to an alternative application example of the portable ultrasonic diagnostic device relating to the present invention. FIG. 9 illustrates the moving state of the display body and the keyboard body of the portable ultrasonic diagnostic device relating to the alternative application example; FIG. 9(a) is a side view showing the state changing from the operating position A to the first storage position B, and FIG. 9(b) is a side view showing the state changing from the operating position A to the second storage position C. FIG. 10 illustrates the moving state of the display body and the keyboard body of the portable ultrasonic diagnostic device relating to the alternative application example; FIG. 10 (a) and FIG. 10 (b) are side views showing the state changing from the operating position A to the first storage position B, and FIG. 10(c) is a side view showing the state changing from the operating position A to the second storage position C.

Firstly, FIG. 9 shows the embodiment being an example of the structure in which the arm parts 54 of the keyboard body 50 are not curved. In the present embodiment, the arm parts 54 are not curved and therefore, the first pivot part 100 is provided at a lower position. Then, as shown in FIG. 9(a), it is possible to change the position of the keyboard body 50, from the nearly horizontal position as indicated by the solid line to the vertical position as indicated by the broken line. On the other hand, the display body 80 is provided being adjacent to the rear of the keyboard body 50 in the vertical position. In addition, as shown in FIG. 9(b), it is possible to change the position from the vertical position as indicated by the solid line to the nearly horizontal position as indicated by the broken line. Here, the upper edge part 58 in the front section of the main body 30 is formed in the shape being rounded off, similar to that of the first embodiment.

According to the embodiment as shown in FIG. 9, the structure thereof is basically the same as that of the first embodiment, and therefore, it is possible to obtain the same operation effect. As shown in FIG. 10(a), the first storage position B of the present embodiment has a structure where the keyboard body 50 in the standing position is provided being biased to the front edge of the main body 30. Therefore, when the portable ultrasonic diagnostic device 1 is tried to be carried by holding the handle part 55 in the first storage position B, there is a problem that the rear section of the portable ultrasonic diagnostic device 1 may turn, failing to keep the holding position for easy carriage.

Therefore, as shown in FIG. 10(b), the rotation angle θ is made larger, so as to mitigate the aforementioned problem. In this case, however, when the rotation angle θ becomes larger, the arm parts 54 may be made longer, and therefore there is another problem that the first pivot part 100 may grow in size. In addition, it is also necessary to provide the display body 80 being displaced toward the rear side. Here, if the display body 80 is displaced to the rear side, there is another problem that this makes it difficult to take the second storage position C as shown in FIG. 10(c). On this occasion, this problem may be solved by cutting off a large portion of the upper edge part 58 in the front section of the main body 30. In this case, however, the front section of the main body 30 is shaped to be thinner, and therefore, a manner of mounting inside the main body needs some contrivance.

As discussed, with the embodiment as shown in FIG. 9 and FIG. 10, it is also possible to obtain the same operation effect as that of the first embodiment, but it is more advantageous for usability to provide the arm parts 54 in the curved shape.

### Explanation of References

1 ... PORTABLE ULTRASONIC DIAGNOSTIC DEVICE, 10 ... ULTRASOUND PROBE, 11 ... ULTRASOUND TRANSMITTER/RECEIVER, 12 ... PROBE CONNECTOR, 13 ... DSC, 14 ... MEMORY UNIT, 15 ... DISPLAY UNIT, 16 ... INPUT UNIT, 17 ... CONTROLLER, 18 ... AUXILIARY EQUIPMENT, 19 ... POWER UNIT, 20 ... CORD CONNECTION PART, 30 ... MAIN BODY, 50 ... KEYBOARD BODY, 51 ... INPUT OPERATION KEY LAYOUT SURFACE, 52 ... TRACKBALL, 53 ... KEYBOARD PART, 54 ... ARM PARTS, 55 ... HANDLE PART, 56 ... OPERATION KEY GROUP, 57 ... PALM REST PART, 58 ... UPPER EDGE PART, 80 ... DISPLAY BODY, 81 ... DISPLAY SCREEN, 82 ... DIVISION PART, 100 ... FIRST PIVOT PART, 200 ... SECOND PIVOT PART, 201 ... PIVOT MAIN BODY, 202 ... DISC-LIKE PART, 203 ... HORIZONTAL PIVOT PARTS, 204 ... VERTICAL PIVOT PART, 250 ... ACCESSORY MOUNT, 251 ... HOOK, A ... OPERATING POSITION, B ... FIRST STORAGE POSITION, C ... SECOND STORAGE POSITION, H ... HEIGHT DIMENSION, D ... DEPTH DIMENSION, W ... WIDTH DIMENSION, E ... GAP, θ ... ROTATION ANGLE, G ... CENTER OF GRAVITY, P ... PIVOT, P1 ... PIVOT, P2 ... PIVOT, P3 ... PIVOT

## Claims

1. A portable ultrasonic diagnostic device comprising a main body, a keyboard body, and a display body, wherein,
the keyboard body comprises an input operation key layout surface on a top face thereof, being coupled to the main body in a rotatable manner via a first pivot part provided on a rear end of the keyboard body, and
the display body comprises a display screen on a front face thereof, being coupled to the main body in a rotatable manner via a second pivot part provided on a lower end of the display body.

2. The portable ultrasonic diagnostic device according to claim 1, wherein,
a pivot of the first pivot part and a pivot of the second pivot part are disposed at an identical pivot.

3. The portable ultrasonic diagnostic device according to claim 1 or claim 2, wherein,
the rear end of the keyboard part comprises arm parts provided with the first pivot part, and the arm parts are formed, being curved with respect to the input operation key layout surface, in such a manner as standing up from a rear section of the keyboard body.

4. The portable ultrasonic diagnostic device according to any one of claim 1 to claim 3, further comprising a handle part in a front section of the keyboard body.

5. The portable ultrasonic diagnostic device according to any one of claim 1 to claim 3, wherein,
the second pivot part comprises a pivot for allowing the display body to swivel.

6. The portable ultrasonic diagnostic device according to claim 5, further comprising an accessory mount, one end thereof being rotatably mounted on a pivot of the second pivot part, the other end being extended toward a rear section of the main body, and the other end is made to stand within a projected area of the main body, wherein,
an accessory hook is provided on the standing end of the accessory mount.

7. The portable ultrasonic diagnostic device according to claim 1, wherein,
a pivot of the first pivot part and a pivot of the second pivot part are arranged in parallel.

8. The portable ultrasonic diagnostic device according to claim 1, wherein,
the first pivot part holds the keyboard body in such a manner that the keyboard body takes a position standing on a top of the main body, from a position nearly horizontal at a front of the main body, and
the second pivot part holds the display body in such a manner that the display body takes a nearly horizontal position for covering a top of the keyboard being in the nearly horizontal position, from a position standing on a top of the main body.

9. The portable ultrasonic diagnostic device according to claim 1, further comprising a palm rest part which is able to be pulled out from the keyboard body.
